# EUROPEAN PATENT SPECIFICATION

(11) **EP 2 138 571 B1**
(45) Date of publication and mention of the grant of the patent: **12.04.2017**
(21) Application number: 08011629.6
(22) Date of filing: 26.06.2008
(51) Int. Cl.: C12N 5/00

(54) **Process for the preparation of multicellular spheroids**
Verfahren zur Herstellung mehrzelliger Sphäroide
Procédé de préparation de sphéroïdes multicellulaires

(43) Date of publication of application: 30.12.2009
(73) Proprietor: SpheroTec GmbH, 82152 Martinsried (DE)
(72) Inventor: Mayer, Barbara, 87700 Memmingen (DE)
(74) Representative: Stolmár & Partner Patentanwälte PartG mbB

(56) References cited:
- WO-A-03/064635
- US-A1- 2003 119 107
- US-A1- 2004 091 460
- US-B1- 6 368 858
- YAMADA K. ET AL.: "Efficient induction of hepatocyte spheroids in a suspension culture using a water-soluble synthetic polymer as an artificial Matrix" JOURNAL OF BIOCHEMISTRY, JAPANESE BIOCHEMICAL SOCIETY / OUP, TOKYO, JP, vol. 123, no. 6, 1 January 1998 (1998-01-01), pages 1017-1023, XP002955752 ISSN: 0021-924X
- PARK K-H: "Improved long-term culture of hepatocytes in a hydrogel containing Arg-Gly-Asp (RGD)" BIOTECHNOLOGY LETTERS, vol. 24, 2002, pages 1131-1135, XP009108470
- VINATIER C. ET AL.: "A silanized hydroxypropyl methylcellulose hydrogel for the three-dimensional culture of chondrocytes" BIOMATERIALS, vol. 26, no. 33, November 2005 (2005-11), pages 6643-6651, XP004989121 ISSN: 0142-9612
- YAMADA K. ET AL.: "Enhanced cell aggregation and liver functions using polymers modified with a cell-specific ligand in primary hepatocyte cultures" JOURNAL OF BIOSCIENCE AND BIOENGINEERING, vol. 88, no. 5, November 1999 (1999-11), pages 557-562, XP002503414 ISSN: 1389-1723
- XU F. ET AL.: "Three-dimensional polymeric systems for cancer cell studies" CYTOTECHNOLOGY, KLUWER ACADEMIC PUBLISHERS, DO, vol. 54, no. 3, 31 July 2007 (2007-07-31), pages 135-143, XP019525901 ISSN: 1573-0778
- MAYER K ET AL: "631 Tumor associated fibroblasts have a profound impact on drug sensitivity of gastric cancer cells", EUROPEAN JOURNAL OF CANCER. SUPPLEMENT, PERGAMON, OXFORD, GB, vol. 2, no. 8, 1 September 2004 (2004-09-01), page 191, XP004640075, ISSN: 1359-6349, DOI: 10.1016/S1359-6349(04)80639-6
- JOKA M ET AL: "prediction of the therapeutic repsonse in colorectal cancer patients using spheroid microtumor technology", JOURNAL OF CLINICAL ONCOLOGY, [Online] vol. 26, no. 15S , page 22178, XP002509449, ISSN: 0732-183X Retrieved from the Internet: URL:http://meeting.ascopubs.org/cgi/conten t/abstract/26/15_suppl/22178> [retrieved on 2009-01-08]

## Description

The invention pertains to a process for the preparation of multicellular spheroids from a suspension of single cells, wherein the cells are directly derived from biological tissue samples and/or cell-containing bodily fluids. The invention is further directed to the multicellular spheroids obtainable by the process according to the invention as well as to the use of the spheroids for diagnostic, screening and therapeutic purposes.

Tissue culturing has traditionally been used as a model for studying disease processes, e.g. cancer, and also for testing potential therapeutic agents for use in the treatment thereof. Generally, cells used for cell cultures are grown into two-dimensional monolayers on plastic plates covered with a liquid medium which supplies essential nutrients and growth factors for the cells. The cells adhere to the bottom surface of the container, assume a characteristic flattened pattern during spreading, and replicate on that surface as a single layer called a monolayer. The media remains on the top of the flat layer of cells and is changed periodically to provide the growing cells with essential nutrients.

An advantage of this system is that the cells are supplied uniformly with the nutrients. Additionally, while the cells are in culture, various agents can be applied to the media in the plates and the effect on the cells can be observed. For example, suspected carcinogens can be added to individual cultures of cells to ascertain if the carcinogen causes the cells to exhibit a growth pattern characteristic of cancerous cells. The analysis of results can be easily carried out, for example by the use of genetic analysis, chromosome analysis or DNA microarray analysis.

Another possibility is to use a cancer cell line to test the effect of different chemotherapeutics on the cells, thus obtaining information about whether a drug is likely to be useful in a therapeutic regimen for the treatment of cancer.

The two-dimensional cultures are often used for replication of cell lines. When it is desired to split the cultures, an enzyme such as trypsin is utilized to destroy the anchorage of the cells to the dish so that subcultures can be made.

Even though two-dimensional monolayer tissue culture has provided great benefits to scientists and clinicians, it suffers from a lingering disadvantage as well. Cells, such as tumor cells, do not grow two-dimensionally in the body and, therefore, whilst monolayer cultures of cells may reflect the architecture of normal organs, such cultures do not reflect the true *in vivo* three-dimensional architecture of tumors.

Because all cells in a monolayer system are subjected to the same growth conditions, this leads to some disadvantages. Namely the resulting culture represents a homogenous cell population wherein every cell is substantially similar to every other cell in the culture. In contrast, naturally occurring cells generally represent a heterogeneous cell population resulting, for example, from positional cues, cell differentiation induced by differences in cellular interactions and the biochemical environment such as hormones, growth factors, oxygen tension, etc.

In an attempt to mimic the conditions in which cells develop *in vivo,* three-dimensional cell culture systems have been developed and used for decades in medical and biologic research. In 1944, first experiments with respect to morphogenesis of amphibian embryos were performed in a three-dimensional cell culture (J. Holtfreter, A study of the mechanics of gastrulation, J. Exp. Zool., 1944, 95: 171-212). Also, embryonic cells (A. Moscana, Cell suspensions from organ rudiments of chick embryos, Exp. Cell Res., 1952, 3: 535-539) and *ex vivo* tumor cells (A. Moscana, The development in vitro of chimeric aggregates of dissociated embryonic chick and mouse cells, Proc. Natl. Acad. Sci. (USA), 1957, 43: 184-104) have been used.

Nowadays, it is preferable to use well-established cell lines since this allows standardization and thus comparability of the results between experiments and laboratories.

To prepare and cultivate cell cultures that mimic the *in vivo* three-dimensional tissue architecture, a number of methods have been developed. These include the spinner flask technique, the liquid-overlay technique, the high aspect rotating vessel technique and the hanging drop method to name but a few.

Generally, these methods involve cultivation of adherent growing cells in vessels with a non-adherent surface, wherein cell aggregation is either induced through movement or wherein single cells are grown into colonies in soft agar or utilizing collagen, fibronectin, laminin or other molecules derived from the extracellular cell matrix (ECM).

WO 95/34637 discloses a method for inducing expression of biomarkers for urologic cancers, including prostate and bladder cancer. Tumor cells are cultured using a three-dimensional technique under conditions effective to induce said expression. The method can be used for diagnostic and therapeutic applications.

A three-dimensional cell culture preparation method is also disclosed in WO 2004/101743 A2 and WO 2005/095585 A1.

Also known in the prior art is a process wherein rat tissue is treated with collagenase before the preparation of the hepatocytes suspension (K. Yamada, Efficient induction of hepatocyte spheroids in a suspension culture using a water-soluble synthetic polymer as an artificial matrix. J. Biochem, 1998 123(6): 1017-1023).

Other methods for cultivation of hepatocytes were known (K-H. Park, Improved long-term culture of hepatocytes in a hydrogel containing Arg-Gly-Asp (RGD), Biotech. Lett., 2002, 24: 1131-1135.)

Known prior to the invention was an injectable and self-setting hydrogel consisting of hydroxypropyl methylcellulose grafted with silanol groups (Si-HPMC) (C. Vinatier, A silanized hydroxypropyl methylcellulose hydrogel for the three dimensional culture of chondrocytes, Biomaterials, 2005, 26(33): 6651).

(K. Yamada, Enhanced cell aggregation and liver functions using polymers modified with a cell-specific ligand in primary hepatocyte culture, J. Bioscience Bioeng., 1999, 88(5): 557-562)

A study was published investigating the impact of benign and tumour associated fibroblasts on drug sensitivity of gastric cancer cells as well as its results, using co-culture experiments of fibroblast cultures and gastric cancer cell lines (K. Mayer, 631 Tumor associated fibroblasts have a profound impact on drug sensitivity of gastric cancer cells, Europ. J. Cancer Suppl., 2004, 2(8): 191).

Also disclosed was the prediction of the therapeutic response in colorectal cancer patients using a spheroid micro tumour technology, but no directly-derived spheroids (M. Joka, Prediction of the therapeutic response in colorectal cancer patients using spheroid microtumor technology, J. Clin. Onc., 2008, 26(15S): 22178).

Lastly, a method for the preparation of multicellular spheroids based on a gel as growth matrix was disclosed by US 6,368,858 B1.

Although the three-dimensional cell culture systems of the prior art represent a valuable transition from the two-dimensional cell culture towards mimicking the *in vivo* cell system, the three-dimensional systems of the prior art continue to suffer from a number of disadvantages with the result that they are still too far removed from the *in vivo* system.

Thus, there is a need for cell culture systems and methods that more effectively mimic the three dimensional cellular relationships and environment of cells *in vivo.*

### Summary of the invention

The present invention provides a process for the preparation of multicellular spheroids comprising
a) generating, in a liquid medium, a suspension of single cells from a biological tissue sample and/or cell containing bodily fluid,
b) adjusting the concentration of cells in the single cell suspension within the range of from 10⁵ to 10⁷ cells/ml liquid medium,
c) adding 2 to 50 vol.-% of an inert matrix to the suspension of single cells,
d) incubating the suspension of single cells,
characterized in that the suspension of single cells is directly derived from at least one biological tissue sample and/or from at least one cell-containing bodily fluid.

Surprisingly it has been found that multicellular spheroids obtained according to the process of the present invention, exhibit an antigen and genetic profile which substantially mimics that of the cells of the biological tissue and/or cell-containing bodily fluid of origin.

Thus, the multicellular spheroids according to the present invention are an ideal system for studying e.g. the effect(s) of chemical compounds such as drugs on the cells. The multicellular spheroids according to the present invention are particularly useful, for example, in diagnosis or determination/suggestion of therapeutic strategies, pharmacokinetic profiling, pharmacodynamic profiling, identification and circumvention of therapeutic resistance, investigations into treatment strategies such as chemotherapy, radiotherapy, hyperthermia or molecular targeted therapies, biomarker identification, tumor profiling, personalised or tailored therapies, testing and/or identification of small molecules, therapeutic proteins and scaffolds, RNA and DNA 'drugs', drug penetration studies and antibody generation.

### Detailed description of the invention

Throughout this application, various articles and patents are referenced. Disclosures of these applications in their entirety are hereby incorporated by reference into this application.

As used herein, the term three-dimensional cell culture refers to any method usable to effect the growth of cells in a three-dimensional multicellular form such as spheroids.

As used herein, the term "spheroid" refers to an aggregate, cluster or assembly of cells cultured to allow three-dimensional growth in contrast to the two-dimensional growth of cells in either a monolayer or cell suspension (cultured under conditions wherein the potential for cells to aggregate is limited). The aggregate may be highly organized with a well defined morphology or it may be a mass of cells that have clustered or adhered together with little organisation reflecting the tissue of origin. It may comprise a single cell type (homotypic) or more than one cell type (heterotypic). Preferably the cells are primary isolates but may also include a combination of primary isolates with an established cell line(s). Particular cell `types' include somatic cells, stem cells, progenitor cells and cancer stem cells.

As used herein, the term "directly derived" refers to a suspension of single cells from a biological tissue and/or cell containing bodily fluid that has been obtained directly from an individual, donor patient or animal without intermediate steps of subculture through a series of cultures and/or hosts. Thus, a suspension of single cells is produced directly from the biological tissue and/or cell-containing bodily fluid. This is in contrast to established methods in which stable and highly passaged cell lines are used. Such cell lines are far removed from being directly derived from their progenitor tissue by several, often a great many, intermediate culture steps. By way of non-limiting example, sources of suitable tissues include benign or malignant primary and metastatic tissues, sources of suitable cell containing bodily fluids include pleural effusion fluid or ascites fluid (liquid tumors).

A "primary culture" is an initial culture of cells freshly isolated from a tissue.

The term "cell line" as used herein refers to cells derived from a primary culture by subculturing and that have exceeded the Hayflick limit. The Hayflick limit may be defined as the number of cell divisions that occur before a cell line becomes senescent or unable to replicate further. This limit is approximately 50 divisions for most non-immortalized cells and in terms of cell culture, equates to approximately 9 to 10 passages of cell subculture over the course of from about 12 to 14 weeks.

Primary tumors are tumors from the original site where they first developed. For example, a primary brain tumor is one that arose in the brain. This is in contrast to a metastatic tumor that arises elsewhere and metastasized or spread to, for example, the brain.

According to the invention the tissue sample which may be used for spheroid preparation may be a normal or healthy biological tissue sample, or may be a biological tissue sample afflicted with a disease or illness, such as a tissue or fluid derived from a tumor. Preferably the tissue sample is a mammalian tissue sample. Also encompassed are metastatic cells. The tissue sample may be obtained from a human, for example from a patient during a clinical surgery or from biopsies. The tissue sample may also be obtained from animals such as mice, rats, rabbits, and the like. It is also possible according to the invention to prepare spheroids from stem cells, progenitor cells or cancer stem cells.

Besides cells originating from tumor tissue, other cells with various indications such as smooth muscle cells, adipocytes, neural cells, stem cells, islet cells, foam cells, fibroblasts, hepatocytes and bone marrow cells, cardiomyocytes and enterocytes are also encompassed within the present invention.

Also within the scope of the present invention is the possibility to rebuild a metastatic microtumor e.g. tumor cells with hepatocytes, or tumor cells with bone marrow cells.

Also useful within the invention are primary cancer cells such as gastric, colon and breast primary cancer cells and metastatic cells. Also encompassed by the invention are primary normal (healthy) cells such as endothelial cells, fibroblasts, liver cells, and bone marrow cells.

Preferably the cells are directly derived from the tissue sample of a patient or healthy donor, a tissue sample derived from a biopsy, surgical specimens, an aspiration or a drainage and also cells from cell-containing bodily fluids.

The prior art methods disclose preparation of spheroids from well known cell lines which may be proliferated multiple times. As a result, the cell lines used in the prior art represent homogeneous cell lines which are not able to mimic a more heterogeneous *in* vivo cell system. Thus, the process of the present invention represents a significant step forward over the prior art since it has previously been extremely difficult to produce spheroids reliably and in useful quantities from "directly derived" or primary isolate samples.

Also within the scope of the invention are large spheroids which consist of a higher cell number in the range of from 10⁶ to 5x10⁶ cells. Large spheroids generally have a necrotic/apoptotic centre that correlates with the upregulation of various biomarkers such as HIF-1alpha, VEGF, TKTL-1 and others. Large and small spheroids are generally used for different purposes, for example, large spheroids may be used as a model of advanced tumors.

The present invention comprises a process for the preparation of multicellular spheroids. In a first step of the method a suspension of single cells is generated from at least one biological tissue sample and/or cell-containing bodily fluid in a medium. The concentration of cells in the suspension is adjusted in the range of from 10⁵ to 10⁷ cells/ml liquid medium. 2 to 50 vol.-% of an inert matrix is then added to the suspension of single cells, which is then incubated, preferably in the presence of CO₂. The process is characterized in that the suspension of single cells is directly derived from at least one biological tissue sample and/or from at least one cell-containing bodily fluid.

In the process according to the invention the cells of the biological tissue sample and/or cell containing bodily fluid are first dissociated or separated from each other. Dissociation of the tissue sample is accomplished by any conventional means known to those skilled in the art. Preferably the tissue sample is treated mechanically or chemically, such as by treatment with enzymes. More preferably the tissue sample is treated both mechanically and enzymatically. Use of the term 'mechanically' means that the tissue is treated to disrupt the connections between associated cells, for example, using a scalpel or scissors or by using a machine, such as a homogenizer. Use of the term 'enzymatically' means that the tissue is treated using one or more enzymes to disrupt the connections between associated cells, for example, by using one or more enzymes such as collagenase, dispases, DNAse and/or hyaluronidase. Preferably a cocktail of enzymes is used under different reaction conditions, such as by incubation at 37°C in a water bath or at room temperature with shaking.

The dissociated tissue is then suspended in a medium to produce a suspension of single cells and from which the spheroids can be formed directly. It should be noted that prior art methods generally include a step of two-dimensional tissue culture in a growth medium prior to attempting three-dimensional cell cultivation. In two-dimensional culturing methods the cell culture adheres to the bottom of a vessel and is then removed for example with Trypsin, EDTA, Bionase or other suitable agents. After this 2D culture step, the suspension of single cells is prepared and from which the spheroids are produced. Thus, the two-dimensional culturing according to the prior art leads to a more or less homogenous cell culture which is accordingly not able to mimic a heterogeneous *in vivo* cell system.

In contrast thereto, it has surprisingly been found that spheroids produced from suspensions of single cells prepared from primary isolate tissue according to the present invention retain essentially all of the biological properties of the originating biological tissue. This is the case for both homotypic and heterotypic cell systems. The same applies when cell-containing bodily fluids are used.

Preferably the suspension of single cells is treated to remove dead and/or dying cells and/or cell debris. The removal of such dead and/or dying cells is accomplished by any conventional means known to those skilled in the art for example, using beads and/or antibody methods. It is known, for example, that phosphatidylserine is redistributed from the inner to the outer plasma membrane leaflet in apoptotic or dead cells. Annexin V and any of its conjugates which have a high affinity for phosphatidylserine can therefore be bound to these apoptotic or dead cells. The use of Annexin V-Biotin binding followed by binding of the biotin to streptavidin magnetic beads enables separation of apoptotic cells from living cells. Other suitable methods will be apparent to the skilled artisan. Surprisingly it has been found that as a result of the inclusion of this step, substantially all of the cells within the suspension of single cells are available to form spheroids with a biological profile or composition more closely mimicking that found *in vivo.*

Methods of the prior art often utilize a dye exclusion test to monitor the vitality or viability of cells. The dye exclusion test is used to determine the number of viable cells present in a cell suspension. It is based on the principle that live cells possess intact cell membranes that exclude certain dyes, such as trypan blue, eosin, or propidium iodide, whereas dead cells do not. In the trypan blue test, a cell suspension is simply mixed with dye and then visually examined to determine whether cells take up or exclude dye. A viable cell will have a clear cytoplasm whereas a nonviable cell will have a blue cytoplasm. Dye exclusion is a simple and rapid technique measuring cell viability but it is subject to the problem that viability is being determined indirectly from cell membrane integrity. Thus, it is possible that a cell's viability may have been compromised (as measured by capacity to grow or function) even though its membrane integrity is (at least transiently) maintained. Conversely, cell membrane integrity may be abnormal yet the cell may be able to repair itself and become fully viable. Another potential problem is that because dye uptake is assessed subjectively, small amounts of dye uptake indicative of cell injury may go unnoticed. In this regard, dye exclusion performed with a fluorescent dye using a fluorescence microscope may result in the scoring of more nonviable cells with dye uptake than tests performed with trypan blue using a transmission microscope. As a result of the use of this method, the suspensions of single cells and spheroids of the prior art comprise a far greater proportion of apoptotic or dead cells. This inclusion of dead matter means that the prior art spheroids are less able to mimic the conditions found in biological tissue *in vivo.*

A more sophisticated method of measuring cell viability is to determine the cell's light scatter characteristics, 7AAD or propidium iodide uptake. It will be apparent to one skilled in the art that use of a flow cytometer coupled with cell sorting may also accomplish removal of dead and/or apoptotic cells.

The suspension of single cells is generated in a liquid culture medium. The medium is designed such that it is able to provide those components that are necessary for the cell's survival. Preferably the suspension of single cells is generated in a liquid medium comprising one or more of the following components: serum, buffer, interleukins, chemokines, growth factors, hydrogen carbonate, glucose, physiological salts, amino acids and hormones.

A preferred medium is RPMI 1640. RPMI 1640 was developed by Moore et. al. at Roswell Park Memorial Institute (hence the acronym RPMI). The formulation is based on the RPMI-1630 series of media utilizing a bicarbonate buffering system and alterations in the amounts of amino acids and vitamins. RPMI 1640 medium has been used for the culture of human normal and neoplastic leukocytes. RPMI 1640, when properly supplemented, has demonstrated wide applicability for supporting growth of many types of cultured cells.

Preferably, the liquid medium further comprises L-glutamine, in particular a stabilized L-glutamine. L-glutamine is an essential nutrient in cell cultures for energy production as well as protein and nucleic acid synthesis. However, L-glutamine in cell culture media may spontaneously degrade, forming ammonia as a by-product. Ammonia is toxic to cells and can affect protein glycosylation and cell viability, lowering protein production and changing glycosylation patterns. It is thus preferred that the L-glutamine is a stabilized glutamine, most preferably it is the dipeptide L-alanyl-L-glutamine, which prevents degradation and ammonia build-up even during long-term cultures. The dipeptide is commercially available as GlutamaxI®.

The liquid medium may further comprise additional components such as antibiotics, for example, penicillin, streptomycin, neomycin, ampicillin, metronidazole, ciprofloxacin, gentamicin, Amphotericin B, Kanamycin, Nystatin; amino acids such as methionine or thymidine; FCS and the like.

In addition to, or instead of, RPMI1640 other liquid media can be used, for example DMEM high or low glucose, Ham's F-10, McCOY's 5A, F-15, RPMI high or low glucose, Medium 199 with Earle's Salts or the different variants of MEM Medium.

In a next step of the method, the concentration of cells in the suspension is adjusted to an appropriate cell concentration. An appropriate cell concentration means an amount of cells per millilitre of culture medium which supports the formation of spheroids in the incubation step. Appropriate cell amounts are 10⁵ to 10⁷ cells/ml liquid medium, preferably 10⁵ to 10⁶ cells/ml medium. Methods of determining cell concentration are known in the art, for example, the cells may be counted with a Neubauer counter chamber (hemocytometer).

In a next step of the process of the present invention an appropriate amount of an inert matrix is added to the suspension of single cells. Use of the term "inert" as used herein refers to a matrix that has limited or no ability to react chemically and/or biologically, i.e. having little or no effect on the biological behaviour or activity of the cells in the suspension. Ideally the inert matrix is of non-human origin.

Preferably the inert matrix increases the viscosity of the culture medium. Not wishing to be bound by theory, it is believed that increasing the viscosity of the culture fluid increases the co-incidental collision and adherence of cells with each other resulting in the formation of aggregates. This is particularly useful since it improves the ability of shear sensitive or weakly adherent cells to aggregate and develop into spheroids.

Thus, the inert matrix supports or promotes the formation of spheroids during the incubation step. Preferably the inert matrix is added to the culture medium in an amount of 2 to 50 vol.-% based on the total volume of the medium. Preferably the inert matrix is added in an amount of 5 to 30 vol.-%, most preferably in an amount of 10 to 15 viol.%. Particular amounts will vary depending on the source or composition of the cells such as 3, 4 or 5 viol.% up to 10 or 15 vol.-% for cell lines and up to 30 to 45 or 50 vol.% when using primary isolate tissue. These amounts are based on the total volume of the medium. The inert matrix is preferably a non-ionic poly(ethylene oxide) polymer, water soluble resin or water soluble polymer such as a cellulose ether. Preferably the inert matrix is selected from the group comprising carboxymethyl cellulose, hydroxypropyl cellulose, hydroxypropylmethyl cellulose, hypomellose, methyl cellulose, methylethyl cellulose. However, also suitable is cellulose, agarose, seaplaque agarose, starch, tragacanth, guar gum, xanthan gum, polyethylene glycol, and the like.

In the next process step of the present invention the single cell suspension is incubated, preferably in the presence of CO₂. Incubation can also be carried out in the presence of water vapour. Possible preparation techniques are e.g. the liquid-overlay technique, the spinner flask technique, the high aspect rotating vessel (HARV) technique or the hanging drop method. These methods are known to the skilled artisan. The HARV technique is inter alia disclosed in US patents 5 153 131, 5 153 132, 5 153 133, 5 155 034, and 5 155 035. The spinner flask technique is disclosed in e.g. W. Mueller-Klieser, "Multicellular Spheroids", J. Cancer Res. Clin. Oncol., 12: 101-122, 1986. The liquid-overlay technique is disclosed e.g. in J.M. Yuhas et.al., "A simplified method for production and growth of multicellular tumor spheroids", Cancer. Res. 37: 3639-3643, 1977. The hanging drop method is disclosed in e.g. Bulletin of Experimental Biology and Medicine, Vol. 91, 3, 1981, Springer, New York. Most preferred in the present invention is the liquid-overlay technique. Generally these preparation techniques are all performed under CO₂ conditions.

The incubation may be performed at 30 to 45°C, preferably at 37 °C, in a normoxic atmosphere containing about 4 to 6 vol.-% CO₂, preferably 5 vol.% CO₂ or under hypoxic conditions, i.e. N₂ 92-95%, O₂ 5-8% . The incubation is performed from about 5 hours to 9 days, preferably of from about 12 hours to 6 days, most preferred of from about 24 to about 96 hours. However, it will be apparent to the skilled artisan that such temperatures and conditions will depend on the source and type of cells used.

As used herein, the term "homotypic' refers to cells of a single type. For example, commercially available cell lines are generally homotypic. In contrast and as used herein, the term 'heterotypic' refers to cells of more than one cell type. For example, primary isolate tissue comprising different cell types will be heterotypic.

Thus, a homotypic spheroid could be prepared from one particular cancer cell line such as Hs746T, malignant primary cells isolated from primary or metastatic malignant tissues or healthy, benign primary cells such as fibroblasts, bone marrow cells, hepatocytes, other benign epithelial cells or chondrocytes. Heterotypic spheroids could be prepared from primary and metastatic patient tissue or from a combination of two or more homotypic cell lines such as a cancer cell line and benign primary cells.

Therefore, in another aspect of the present invention, a suspension of single homotypic cells may be combined with, for example, epithelial cells, immune cells, fibroblasts, hepatocytes, chondrocytes, bone marrow cells, airway epithelial cell cultures, enterocytes, cardiomyocytes, melanocytes, keratinocytes, adipocytes, stem cells, cancer stem cells and/or smooth muscle cells, resulting in the formation of heterotypic spheroids. It will be apparent to one skilled in the art that a suspension of single heterotypic cells may also be combined in this manner.

The advantage of combining homotypic cell types with other cell types, such as epithelial cells, immune cells, fibroblasts (as well as other cell types cited above), is that tumour cells interact with epithelial cells, immune cells and/or fibroblasts (and also with such cells cited above) in nature. Hence, the combination with such cells leads to a heterotypic, multicellular spheroid system which mimics even more closely an *in vivo* cell or metastic cell system.

The internal environment of a spheroid is dictated by the metabolism and adaptive responses of cells with a well-defined morphological and physiological geometry. Most homotypic spheroids develop concentric layers of heterogeneous cell populations with cells at the periphery and layers of quiescent cells close to a necrotic core. The heterogeneous arrangement of cells in a spheroid mimics initial avascular stages of early tumours. Although homotypic spheroids are able to mimic closely the *in vivo* morphology, some of the biological complexity is lost. Thus, by co-culturing more than one cell type, tumour cell interactions with other cell types reflecting natural cell interaction *in vivo* can be established better representing the *in vivo* environment.

Thus, suspensions of single cells may be combined with other cells, for example from established cell lines, primary cells and/or primary or metastatic tissues. Most preferably the tissue is a tumour tissue wherein the cancer cell lines may be cell lines from gastric (e.g. Hs-746T, MKN-28, N87, and the like), colorectal (e.g. HT-29, HCT-116, DLD-1, and the like), liver (e.g. HepG2, and the like), pancreas (e.g. L.6pl, AsPC-1, MiaPACA, and the like), lung (e.g. A549, H358, H1299, and the like), kidney (e.g. 786-0, A-498, CAKI-1, and the like), breast (e.g. MCF-7, BT549, Hs575T, and the like), cervical (e.g. HeLa, and the like), prostate (e.g. PC-3, LNCaP, DU-145, and the like) or glioma (e.g. U251, U373, and the like) cell lines. It will be appreciated that the method is suitable for use with any cell line. In particular preferred are also cell lines from sarcoma or astrocytoma tissue.

Another aspect of the invention is a multicellular spheroid, which is obtained by the process according to the invention, wherein the multicellular spheroid is an aggregate, cluster or assembly of cells wherein the cells are primary isolates. The spheroid may comprise a single cell type (homotypic) or a mixture of two or more cell types (heterotypic).

The process as set forth above leads to spheroids with a nearly homogenous spherical shape, wherein the average diameter of the spheroids reaches from 50 to 2000 µm, preferably from 150 to 1000 µm and most preferred from 200 to 500 µm.

The multicellular spheroids obtained by the process according to the invention can also be characterised in that they exhibit characteristics that substantially mimic those of the tissue of origin, such as: antigen profile and/or genetic profile, tumour biologic characteristics, tumour architecture, cell proliferation rate(s), tumour microenvironments, therapeutic resistance and composition of cell types. Preferably, they exhibit an antigen profile and genetic profile which is substantially identical to that of the tissue of origin.

Thus, the spheroids of the invention exhibit a substantially similar/identical behaviour to that of natural cell systems, e.g. with respect to organization, growth, viability, cell survival, cell death, metabolic and mitochondrial status, oxidative stress and radiation response as well as drug response.

Since the multicellular spheroids according to the invention are substantially identical to *in vivo* cell systems, these spheroids can thus be used for diagnostic and/or therapeutic purposes, for example, pharmacokinetic profiling, pharmacodynamic profiling, efficacy studies, cytotoxicity studies, penetration studies of compounds, therapeutic resistance studies, antibody generation, personalized or tailored therapies, RNA/DNA 'drug' testing, small molecule identification and/or testing, biomarker identification, tumour profiling, hyperthermia studies, radioresistance studies and the like.

In one aspect, the multicellular spheroids can be obtained from benign or malignant tissues or from primary cells and used for the screening of compounds, for example, as new therapeutic agents or screening for e.g. chemotherapeutica wherein the response of the spheroid to the chemotherapeuticum can be determined. It is thus possible to see whether a chemotherapeuticum has an effect and/or side effects on the multicellular spheroid, e.g. whether it causes cell death (apoptosis) or other biologic effect.

In the sense of the present invention, preferably the term "chemotherapeutica" should be understood as to include all chemical substances used to treat disease. More particularly, it refers to antineoplastic drugs used to treat cancer or the combination of these drugs into a standardized treatment regimen. In its non-oncological use, the term may also refer to antibiotics (antibacterial chemotherapy). Other uses of cytostatic chemotherapy agents are the treatment of autoimmune diseases such as multiple sclerosis and rheumatoid arthritis, viral infections, heart diseases and the suppression of transplant rejections. It will of course be apparent to the skilled artisan that such chemotherapeutica need not be limited to substances used to treat disease. Thus, the term may be applied more loosely to refer to any agent that the skilled person wishes to expose the spheroids to determine whether said agent has an effect, for example, on the behaviour or biological characteristics of the spheroids.

By way of non-limiting example, chemotherapeutic agents may include: alkylating agents, antimetabolites, anthracyclines, plant alkaloids, topoisomerase inhibitors, and other antitumour agents, antibodies such as monoclonal, single chain or fragments thereof and the new tyrosine kinase inhibitors e.g. imatinib mesylate (Gleevec® or Glivec®), small molecules, tyrosine kinase receptor inhibitors, anticalins, aptamers, peptides, scaffolds, biosimilars, generic drugs, siRNA and RNA or DNA based agents.

### Examples

### Example 1: Preparation of heterotypic spheroids derived from primary patient tumour tissue.

A gastric cancer tissue with a size of about 0.5 cm³ was taken from a patient. The tissue was made into a suspension of single cells by reducing the tissue into small pieces with the aid of a scalpel and subsequent treatment with an enzyme cocktail consisting of collagenase, dispase, DNAse and hyaluronidase. Then, the cells were suspended in RPMI 1640 culture medium containing Glutamax I™ or L-Glutamine.

The viability of the cells was tested with the trypan-blue exclusion test and the cell number was adjusted to 10⁶ cells/ml medium with the aid of a Neubauer counter chamber. Cellulose ether was then added to the cell suspension and the suspension transferred to a 96-well plate with the following amounts of reagents:
For each well plate 12 ml cell suspensions were prepared (96 well x 100 µl/well = -10 ml + 2 ml excess = 12 ml), to provide a concentration of cells of 5 x 10⁴ cells/100 µl medium corresponding to 5 x 10⁵/ml corresponding to 6 x 10⁶/ 12ml.

The final suspension contained 6 ml of cell suspension, 5,5 ml RMPI 1640 + Glutamax™ and 0,6 ml cellulose ether (=5%)

The cell suspension was mixed and then transferred with a multichannel pipette to a 96-well plate in an amount of 100 µl/well. The cell suspension was then incubated at 37 °C in the presence of 5 % CO₂ for 24 hours.

After 24 hours, multicellular spheroids had formed and exhibited a homogeneous shape with a mean diameter of about 250 µm.

### Example 2: Preparation of heterotypic spheroids by combining a homotypic cell line with a primary cell type

Homotypic cells from a human gastric carcinoma cell line (Hs746T) were suspended in RPMI 1640 culture medium containing Glutamax I™ or L-Glutamine.

The viability of the cells was tested with the trypan-blue exclusion test and the concentration of cells was adjusted to 10⁶ cells/ml medium with the aid of a Neubauer counter chamber. Cellulose ether was then added to the cell suspension and the suspension transferred to a 96-well plate with the following amounts of reagents:
For each well plate 12 ml cell suspension was prepared (96 well x 100 µl/well = -10 ml + 2 ml excess = 12 ml), to provide a concentration of cells of 5 x 10⁴ cells/100 µl medium corresponding to 5 x 10⁵/ml corresponding to 6 x 10⁶/ 12ml.

The final suspension contained 6 ml of the cell suspension, 5,5 ml RMPI 1640 + Glutamax™ and 0,6 ml cellulose ether (=5%).

The Hs746T cell suspension was mixed with fibroblasts (in either a 1:1 ratio or 9:1 ratio) and transferred with a multichannel pipette to a 96-well plate in an amount of 100 µl/well. The cell suspension was then put in an incubator and incubated at 37 °C in the presence of 5% CO₂ for 24 hours.

After 24 hours heterotypic multicellular spheroids had formed. The spheroids comprised both cells of the Hs746T cell line and fibroblast cells and exhibited a homogeneous shape with a mean diameter of about 200 µm.

### Example 3: Preparation of heterotypic spheroids by combining a homotypic cell line with two or more different primary cell types, e.g. the gastric cancer cell line Hs746T cocultured with immune cells and hepatocytes

Homotypic cells from a human gastric carcinoma cell line (Hs746T) were suspended in RPMI 1640 culture medium containing Glutamax I™ or L-Glutamine.

The viability of the cells was tested with the trypan-blue exclusion test and the concentration of cells was adjusted to 10⁶ cells/ml medium with the aid of a Neubauer counter chamber. Cellulose ether was then added to the cell suspension and the suspension transferred to a 96-well plate with the following amounts of reagents:
For each well plate 12 ml cell suspension was prepared (96 well x 100 µl/well = ∼10 ml + 2 ml excess = 12 ml), to provide a concentration of cells of 5 x 10⁴ cells/100 µl medium corresponding to 5 x 10⁵/ml corresponding to 6 x 10⁶/12ml.

The final suspension contained 6 ml of the cell suspension, 5,5 ml RMPI 1640 + Glutamax™ and 0,6 ml cellulose ether (=5%).

The Hs746T cell suspension was mixed with two cell types, immune cells and hepatocytes, derived from primary tissues (in either a 1:1 ratio or 9:1 ratio) and transferred with a multichannel pipette to a 96-well plate in an amount of 100 µl/well. The cell suspension was then put in an incubator and incubated at 37 °C in the presence of 5 % CO₂ for 24 hours.

After 24 hours heterotypic multicellular spheroids had formed. The spheroids comprised both cells of the Hs746T cell line and both immune cells and hepatocytes. The heterotypic spheroids exhibited a homogeneous shape with a mean diameter of about 200 µm.

## Claims

1. Process for the preparation of multicellular spheroids comprising
a) generating in a liquid medium, a suspension of single cells from at least one biological tissue sample or cell-containing bodily fluid,
b) adjusting the concentration of cells in the suspension to a concentration in the range of from 10⁵ cells to 10⁷ cells / ml liquid medium,
c) adding 2 to 50 vol.-% of an inert matrix to the suspension of single cells,
d) incubating the suspension of single cells,
**characterised in that** the suspension of single cells is directly derived from at least one biological tissue sample or cell-containing bodily fluid.

2. Process according to claim 1, wherein the tissue sample is either a healthy tissue sample, a tumour tissue sample, or a benign or malignant primary tissue sample.

3. Process according to claim 1 or 2, further comprising the step of a) (ii) treating the suspension of single cells to remove dead and/or dying cells and/or cell debris.

4. Process according to any one of claims 1 to 3, wherein the tissue sample is a mammalian tissue sample.

5. Process according to any one of claims 1 to 4, wherein the tissue sample is treated mechanically and/or enzymatically before generation of the suspension of single cells.

6. Process according to any one of claims 1 to 5, wherein the single cell suspension is generated in the liquid medium comprising serum, buffer, interleukins, chemokines, growth factors, hydrogen carbonate, glucose, physiological salts, amino acids and hormones.

7. Process according to claim 6, wherein the liquid medium further comprises at least one antibiotic selected from the group consisting of penicillin, streptomycin, neomycin, ampicillin, metronidazole, ciprofloxacin, gentamicin, Amphotericin B, Kanamycin and Nystatin.

8. Process according to any one of the preceding claims, wherein the concentration of single cells is adjusted to 10⁵ to 5x10⁶ cells/ml liquid medium.

9. Process according to any one of the preceding claims, wherein the inert matrix is added in an amount of from 5 to 30 vol.-% based on the total volume of the liquid medium.

10. Process according to any one of the preceding claims, wherein the inert matrix is derived from a non-human source.

11. Process according to any one of the preceding claims, wherein the inert matrix is selected from the group comprising cellulose ether, carboxymethyl cellulose, hydroxypropyl cellulose, hydroxypropylmethyl cellulose, hypomellose, methyl cellulose, methylethyl cellulose, polyethylene glycol, and agarose.

12. Process according to any one of the preceding claims, wherein the incubation is performed at 37 °C in an atmosphere containing from 4 to 6 vol.-% CO₂.

13. Process according to any one of the preceding claims, wherein the incubation is performed from 12 hours up to about 9 days.

14. Process according to any one of the preceding claims, wherein the suspension of single cells is combined with at least one cell type selected from the group comprising of epithelial cells, immune cells, hepatocytes, chondrocytes, bone marrow cells, airway epithelial cell culture, melanocytes, keratinocytes, adipocytes, smooth muscle cells, fibroblasts, enterocytes, stem cells, cancer stem cells and cardiomyocytes.

15. Multicellular spheroid, obtained by a process of any one of claims 1 to 13, wherein the multicellular spheroid is an aggregate, cluster or assembly of cells wherein the cells are primary isolates.

16. Multicellular spheroid according to claim 15, wherein the at least one biological tissue is generated from benign or malignant primary or metastatic tissue.

17. Multicellular spheroid according to claim 15 or 16, **characterized by** a homogeneous spherical shape with an average diameter of from 50 to 2000 µm.

18. Multicellular spheroid according to any one of claims 15 to 17, wherein at least one of the antigen profile, genetic profile, tumour biologic characteristics, tumour architecture, cell proliferation rate(s), tumour microenvironments, therapeutic resistance or composition of cell types is substantially identical to that of the tissue of origin.

19. Multicellular spheroid according to any one of claims 15 to 17, wherein the antigen profile and the genetic profile of the spheroid is substantially identical to the antigen profile and the genetic profile of the tissue of origin.

20. Use of a multicellular spheroid according to any one of claims 15 to 18 or obtained by a process according to any one of claims 1 to 14 for screening compounds for diagnostic and/or therapeutic purposes, pharmacokinetic profiling, pharmacodynamic profiling, efficacy studies, cell toxicity studies, drug penetration studies, biomarker identification and/or tumour profiling and therapeutic resistance studies.

## Patentansprüche

1. Verfahren zur Herstellung von multizellulären Spheroiden, umfassend:
a. Erzeugen, in einem flüssigen Medium, einer Suspension von Einzelteilen von mindestens einer biologischen Gewebeprobe oder zellenthaltenden Körperflüssigkeit,
b. Anpassen der Konzentration von Zellen in der Suspension auf eine Konzentration im Bereich von 10⁵ bis 10⁷ Zellen/ml flüssiges Medium,
c. Hinzufügen von 2-50 Vol.% einer inerten Matrix zur Suspension von Einzelzellen,
d. Inkubieren der Suspension von Einzelzellen,
**dadurch gekennzeichnet, dass** die Suspension von Einzelzellen direkt von mindestens einer biologischen Gewebeprobe oder zellenthaltenden Körperflüssigkeit gewonnen wird.

2. Verfahren gemäß Anspruch 1, wobei die Gewebeprobe entweder eine gesunde Gewebeprobe, eine Tumorgewebeprobe, oder eine gutartige oder bösartige primäre Gewebeprobe ist.

3. Verfahren gemäß Anspruch 1 oder 2, weiter umfassend den Schritt des a. (ii) Behandelns der Suspension von Einzelzellen, um tote und/oder sterbende Zellen und/oder Zelltrümmer zu entfernen.

4. Verfahren gemäß einem der Ansprüche 1-3, wobei die Gewebeprobe eine Säugetier-Gewebeprobe ist.

5. Verfahren gemäß einem der Ansprüche 1-4, wobei die Gewebeprobe vor Erzeugen der Suspension von Einzelzellen mechanisch und/oder enzymatisch behandelt wird.

6. Verfahren gemäß einem der Ansprüche 1-5, wobei die Einzelzellsuspension in einem flüssigen Medium umfassend Serum, Puffer, Interleukine, Chemokine, Wachstumsfaktoren, Hydrogencarbonat, Glucose, physiologische Salze, Aminosäuren und Hormone erzeugt wird.

7. Verfahren gemäß Anspruch 6, wobei das flüssige Medium weiter mindestens ein Antibiotikum ausgewählt aus der Gruppe bestehend aus Penicillin, Streptomycin, Neomycin, Ampicillin, Metronidazol, Ciprofloxacin, Gentamicin, Amphotericin B, Kanamycin und Nystatin enthält.

8. Verfahren gemäß einem der vorhergehenden Ansprüche, wobei die Konzentration an Einzelzellen auf 10⁵ bis 5x10⁶ Zellen/ml flüssiges Medium eingestellt wird.

9. Verfahren gemäß einem der vorhergehenden Ansprüche , wobei die inerte Matrix in einer Menge von 5 bis 30 Vol.% basierend auf dem Gesamtvolumen des flüssigen Mediums hinzugefügt wird.

10. Verfahren gemäß einem der vorhergehenden Ansprüche, wobei die inerte Matrix von einer nicht-menschlichen Quelle gewonnen wird.

11. Verfahren gemäß einem der vorhergehenden Ansprüche, wobei die inerte Matrix ausgewählt wird aus der Gruppe umfassend Zelluloseether, Carboxymethylzellulose, Hydroxypropylzellulose, Hydroxypropylmethylzellulose, Hypomellose, Methylzellulose, Methylethylzellulose, Polyethylenglycol und Agarose.

12. Verfahren gemäß einem der vorherigen Ansprüche, wobei die Inkubation bei 37 °C in einer Atmosphäre umfassend 4-6 Vol.% CO₂ durchgeführt wird.

13. Verfahren gemäß einem der vorhergehenden Ansprüche, wobei die Inkubation von 12 Stunden bis zu etwa 9 Tagen durchgeführt wird.

14. Verfahren gemäß einem der vorhergehenden Ansprüche, wobei die Suspension von Einzelzellen kombiniert wird mit mindestens einem Zelltyp ausgewählt aus der Gruppe bestehend aus Epithelzellen, Immunzellen, Hepatozyten, Chondrozyten, Knochenmarkszellen, Epithelzellkultur der Atemwege, Melanozyten, Keratinozyten, Adipocyten, glatte Muskelzellen, Fibroblasten, Enterozyten, Stammzellen, Krebsstammzellen und Kardiomyozyten.

15. Multizelluläres Spheroid, erhalten durch ein Verfahren gemäß einem der Ansprüche 1 bis 13, wobei das multizelluläre Spheroid ein Aggregat, Cluster oder Ansammlung von Zellen ist, wobei die Zellen primäre Isolate sind.

16. Multizelluläres Spheroid gemäß Anspruch 15, wobei mindestens ein biologisches Gewebe aus einem gutartigen oder bösartigen primären oder metastatischen Gewebe erzeugt wird.

17. Multizelluläres Spheroid gemäß einem der Ansprüche 15 oder 16, durch eine homogene sphärische Form mit einem durchschnittlichen Durchmesser von 50 bis 2000 µm gekennzeichnet.

18. Multizelluläres Spheroid gemäß einem der Ansprüche 15 bis 17, wobei mindestens eines von Antigenprofil, genetischem Profil, tumorbiologischen Eigenschaften, Tumorarchitektur, Zellproliferationsrate(n), Tumor-Mikroumgebungen, therapeutische Resistenz oder Zusammensetzung der Zelltypen im Wesentlichen identisch mit dem des Ursprungsgewebes ist.

19. Multizelluläres Spheroid gemäß einem der Ansprüche 15 bis 17, wobei das Antigenprofil und das genetische Profil des Spheroids im Wesentlichen identisch mit dem Antigenprofil und dem genetischen Profil des Ursprungsgewebes sind.

20. Verwendung eines multizellulären Spheroids gemäß einem der Ansprüche 15 bis 18 oder erhalten durch ein Verfahren gemäß einem der Ansprüche 1 bis 14 zum Screening von Verbindungen für diagnostische und/oder therapeutische Zwecke, pharmakokinetisches Profiling, pharmakodynamisches Profiling, Effizienzstudien, Zelltoxizitätsstudien, Medikamenten-Penetrations-Studien, Biomarker-Identifikation und/oder Tumor-Profiling und therapeutische Resistenzstudien.

## Revendications

1. Procédé pour la préparation des sphéroïdes multicellulaires comprenant
a) générer, dans un milieu liquide, une suspension des cellules uniques d'au moins un échantillon de tissu biologique ou d'un fluide corporel comprenant des cellules,
b) ajuster la concentration des cellules dans la suspension à une concentration dans l'intervalle de 10⁵ cellules à 10⁷ cellules/ml milieu liquide,
c) ajouter 2 à 50 vol.-% d'une matrice inerte à la suspension des cellules uniques,
d) incuber la suspension des cellules uniques,
**caractérisé en ce que** la suspension des cellules uniques est dérivée directement d'au moins un échantillon de tissu biologique ou d'un fluide corporel comprenant des cellules.

2. Procédé selon la revendication 1, dans lequel l'échantillon de tissu est soit un échantillon de tissu sain, soit un tissu d'une tumeur, soit un échantillon de tissu primaire bénin ou malin.

3. Procédé selon la revendication 1 ou 2, en outre comprenant l'étape de a) (ii) traiter la suspension des cellules uniques pour enlever des cellules mortes et/ou mourantes et/ou des débris cellulaires.

4. Procédé selon l'une des revendications 1 à 3, dans lequel l'échantillon de tissu est un échantillon de tissu mammalien.

5. Procédé selon l'une des revendications 1 à 4, dans lequel l'échantillon de tissu est traité mécaniquement et/ou enzymatiquement avant la génération de la suspension des cellules uniques.

6. Procédé selon l'une des revendications 1 à 5, dans lequel la suspension des cellules uniques est générée dans un milieu liquide comprenant serum, tampon, interleukines, chimiokines, facteurs de croissance, carbonate d'hydrogène, glucose, sels physiologiques, aminoacides et hormones.

7. Procédé selon la revendication 6, dans lequel le milieu liquide comprend en outre au moins un antibiotique sélectionné du groupe consistant de pénicilline, streptomycine, néomycine, ampicilline, métronidazole, ciprofloxacine, gentamicine, amphotéricine B, kanamycine and nystatine.

8. Procédé selon l'une des revendications précédentes, dans lequel la concentration des cellules uniques est ajustée à 10⁵ à 5x10⁶ cellules/ml milieu.

9. Procédé selon l'une des revendications précédentes, dans lequel la matrice est ajoutée dans une quantité de 5 à 30 vol.-% basé sur le volume total du milieu liquide.

10. Procédé selon l'une des revendications précédentes, dans lequel la matrice inerte est dérivée d'une source non-humaine.

11. Procédé selon l'une des revendications précédentes, dans lequel la matrice inerte est sélectionnée du groupe comprenant éther de cellulose, carboxyméthyle cellulose, hydroxypropyle cellulose, hydroxypropylméthyle cellulose, hypomellose, méthyle cellulose, méthyléthyle cellulose, polyéthylène glycol, et agarose.

12. Procédé selon l'une des revendications précédentes, dans lequel l'incubation est exécutée à 37 °C dans une atmosphère contenant de 4 à 6 vol.-% CO₂.

13. Procédé selon l'une des revendications précédentes, dans lequel l'incubation est exécutée de 12 heures jusqu'à environ 9 jours.

14. Procédé selon l'une des revendications précédentes, dans lequel la suspension des cellules uniques est combinée avec au moins un type de cellule sélectionné du groupe comprenant cellules épithéliales, cellules immunes, hépatocytes, chondrocytes, cellules de la moelle osseuse, culture des cellules épithéliales des voies respiratoires, mélanocytes, kératinocytes, adipocytes, cellules de la musculature lisse, fibroblastes, entérocytes, cellules souche, cellules souche du cancer et cardiomyocytes.

15. Sphéroïde multicellulaire, obtenu par le procédé selon l'une des revendications 1 to 13, dans lequel le sphéroïde est un agrégat, un cluster ou une assemblée des cellules dans lequel les cellules sont des isolats primaires.

16. Sphéroïde multicellulaire selon la revendication 15, dans lequel au moins un tissue biologique est généré d'un tissu primaire bénin ou malin ou d'un tissu métastatique.

17. Sphéroïde multicellulaire selon la revendication 15 ou 16, **caractérisé par** une forme sphérique homogène avec un diamètre moyen de 50 à 2000 µm.

18. Sphéroïde multicellulaire selon l'une des revendications 15 à 17, dans lequel au moins un de profil d'antigènes, profil génétique, caractéristiques biologiques du tumeur, architecture du tumeur, taux de prolifération des cellules, microenvironnements du tumeur, résistance thérapeutique or composition des types des cellules est substantiellement identique au cela du tissu d'origine.

19. Sphéroïde multicellulaire selon l'une des revendications 15 à 17, dans lequel le profil d'antigènes et le profil génétique du sphéroïde sont substantiellement identique au profil d'antigènes et au profil génétique du tissu d'origine.

20. Utilisation d'un sphéroïde multicellulaire selon l'une des revendications 15 à 18 ou obtenu par un procédé selon l'une des revendications 1 à 14 pour la sélection des composés pour des objets diagnostiques ou thérapeutiques, le profilage pharmacocinétique, le profilage pharmacodynamique, les études de l'efficience, les études de la toxicité cellulaire, les études de la pénétration des médicaments, l'identification des marqueurs biologiques et/ou le profilage des tumeurs et les études de la résistance thérapeutique.
